# EUROPÄISCHE PATENTANMELDUNG

(11) **EP 1 254 927 A2**
(43) Veröffentlichungstag der Anmeldung: **06.11.2002**
(21) Anmeldenummer: 02007356.5
(22) Anmeldetag: 08.04.2002
(51) Int. Cl.: C09C 1/00

(54) **Pigmentpräparation**

(30) Priorität: 27.04.2001 DE 10120856
(71) Anmelder: MERCK PATENT GmbH, 64293 Darmstadt (DE)
(72) Erfinder: Weitzel, Joachim, Savannah Gardens, Georgia 31410 (US); Hechler, Wolfgang, 64686 Lautertal (DE)

(57) **Zusammenfassung**

Die vorliegende Erfindung betrifft eine Pigmentzubereitung enthaltend BiOCI-Pigmente, ein oder mehrere Glanzpigmente und sphärische Teilchen sowie deren Verwendung in Druckfarben, Sicherheitsdruckfarben, Lacken, Farben, Kunststoffen und in kosmetischen Formulierungen.

## Beschreibung

Die vorliegende Erfindung betrifft eine Pigmentpräparation im wesentlichen bestehend aus einer Mischung von BiOCI-Pigmenten, Glanzpigmenten und sphärischen Teilchen, sowie deren Verwendung, insbesondere in Druckfarben.

Druckfarben bestehen in der Regel aus Bindemitteln, Pigmenten oder Farbstoffen und Additiven. Bei Druckerzeugnissen für Verpackungsdrucke, Etiketten und hochwertigen Zeitschriften tritt immer häufiger die Forderung auf den abgebildeten Gegenständen einen speziellen Glanz zu verleihen.

Glanzpigmente enthaltende Offset-Druckfarben zeigen alle den Nachteil, dass sie Probleme mit der Fortdruckstabilität aufweisen. Sie neigen dazu auf Farbwerk, Druckplatte und Gummituch schnell aufzubauen oder zu sedimentieren, so dass ein problemloser Fortdruck über 10000 Bögen in der Regel nicht möglich ist. Ein grundsätzliches Problem ist die starke Neigung von Glanzpigmenten, insbesondere Perlglanzpigmenten, bedingt durch die plättchenförmige Struktur sowie der speziellen chemisch/physikalischen Oberflächeneigenschaften zur Bildung von Agglomeraten in der Druckfarbe, wobei die Pigmente stapelförmig aufeinanderliegen und aufgrund von starker Adhäsion nur schwer zu separieren sind. Weiterhin ist die Glanzgebung solcher Andrucke in der Regel nicht zufriedenstellend, was auf die unzureichende Menge an übertragenden Pigmenten auf das Druckerzeugnis zurückzuführen ist. Die Farbe verarmt auf dem Transportweg über Farbwerk, Platte und Gummituch an Pigment. Letzteres reichert sich an exponierten Stellen auf Platte und Tuch an und führt zu Aufbauen und Pelzen.

Pigmentpräparationen mit Perlglanzpigmenten und sphärischen Teilchen sind bereits aus der DE-A-4446456 bekannt. In Publikationen der Firma EM Industries sowie der Firma Mearl Corporation wird die Verwendung von BiOCI-Pigmenten in Druckfarben beschrieben. Perlglanzpigmenthaltige Präparationen für den Offsetdruck weisen einen hohen Glanz auf, besitzen aber den Nachteil, dass die Perlglanzpigmenten beim Verdrucken in Abhängigkeit des verwendeten Bindemittels eine große Tendenz zur Migration in das Feuchtmittel zeigen. BiOCI-Pigmente werden beim Druckvorgang zermahlen, was zur Folge hat, dass die optischen Eigenschaften der Pigmente weitgehend verloren gehen.

Es bestand daher die Aufgabe eine Pigmentzubereitung, vorzugsweise für Druckfarben, insbesondere für Offset-Druckfarben, bereitzustellen, die Glanzpigmente enthält, aber die oben genannten Nachteile nicht aufweist.

Überraschenderweise wurde nun gefunden, dass eine geringere Pigmentmigration in das Feuchtmittel beobachtet wird und die Güte des Glanzeffektes sowie die Menge der übertragenen Pigmentteilchen in der Druckfarbe gesteigert werden kann, wenn man eine Pigmentpräparation verwendet, die neben Glanzpigmenten zusätzlich BiOCI-Pigmente und sphärische Teilchen enthält.

Die erfindungsgemäße Pigmentpräparation weist gegenüber den Offset-Präparationen aus dem Stand der Technik, die nur Perlglanzpigmente bzw. nur BiOCI-Pigmente enthalten, folgende Vorteile auf:
- einen höheren Glanz
- ein geschlosseneres Druckbild
- einen besseren Pigmenttransfer
- eine geringere Pigmentmigration in das Feuchtmittel
- ein besseres Deckvermögen.

Gegenstand der Erfindung ist somit eine Pigmentpräparation enthaltend BiOCI-Pigmente, ein oder mehrere Glanzpigmente, sphärische Teilchen und gegebenenfalls Additive aus dem Druckbereich.

Durch die Kombination von BiOCI-Pigmenten mit Glanzpigmenten lassen sich in den Druckfarben deutlich mehr koloristische Effekte erzielen. Das Verhältnis BiOCI zu Glanzpigment beträgt vorzugsweise 99 : 1 bis 1 : 99 und insbesondere 70 : 30 bis 30 : 70. Die erfindungsgemäße Pigmentpräparation enthält vorzugsweise 40 bis 99,8 Gew.%, insbesondere 70 bis 99,5 Gew.%, an BiOCI- und Glanzpigmenten, bezogen auf die Präparation.

BiOCI-Pigmente für den Offsetdruck sind kommerziell erhältlich, z. B. von der Fa. Engelhard unter dem Namen Mearlite Ultra Fine OFS sowie von der Fa. Merck KGaA unter dem Namen Bi-Flair® 66a. Die im Handel erhältlichen BiOCI-Pigmente besitzen Teilchengrößen von 1-50 µm. Für die erfindungsgemäße Pigmentpräparation sind insbesondere BiOCI-Pigmente mit Teilchengrößen von 5-20 µm geeignet.

Die BiOCI-Pigmente können in der erfindungsgemäßen Pigmentpräparation auch in angeteigter Form eingesetzt werden. Diese Dispersionen verfügen im Vergleich zu pulverförmigen BiOCI-Pigmenten über einen deutlich höheren Silberglanz, höhere Transparenz und sind noch leichter dispergierbar. Insbesondere geeignet sind BiOCI-Anteigungen in Rizinusöl, Nitrocellulose, Mineralöl und Alkydharz.

Weiterer wichtiger Bestandteil der erfindungsgemäßen Pigmentzubereitung ist das Glanzpigment. Die Pigmentzubereitung kann dabei auch ein Gemisch unterschiedlicher Glanzpigmente enthalten um z. B. bestimmte Farbgebungen zu erzielen.

Als Glanzpigmente werden vorzugsweise Pigmente auf der Basis plättchenförmiger, transparenter oder semitransparenter Substrate aus z. B. Schichtsilikaten, wie etwa Glimmer, synthetischen Glimmer, SiO₂-, Glas-, TiO₂-, Al₂O₃-Plättchen, Talkum, Sericit, Kaolin, aus Glas oder anderen silikatischen Materialen verwendet, die mit seltenen Erdmetallsulfiden, wie z. B. Ce₂S₃, mit farbigen oder farblosen Metalloxiden, wie z. B. TiO₂, Titansuboxide, Titanoxinitride, Pseudoprookit, Fe₂O₃, Fe₃O₄, SnO₂, Cr₂O₃, ZnO, CuO, NiO und anderen Metalloxiden allein oder in Mischung in einer einheitlichen Schicht oder in aufeinander folgenden Schichten (Multilayerpigmente) belegt sind. Perlglanzpigmente sind z. B. aus den deutschen Patenten und Patentanmeldungen 14 67 468, 19 59 998, 20 09 566, 22 14 454, 22 15 191, 22 44 298, 23 13 331, 25 22 572, 31 37 808, 31 37 809, 31 51 343, 31 51 354, 31 51 355, 32 11 602, 32 35 017 und P 38 42 330 bekannt und im Handel erhältlich, z. B. unter der Marke Iriodin® der Fa. Merck KGaA, Darmstadt, Deutschland.

Besonders bevorzugte Pigmentpräparationen enthalten mit TiO₂, Fe₂O₃ oder TiO₂/Fe₂O₃ beschichtete Glimmer-, Al₂O₃-, Glas- oder SiO₂-Plättchen. Die Beschichtung der SiO₂-Plättchen kann z. B. erfolgen wie in der WO 93/08237 (nasschemische Beschichtung) oder DE-OS 196 14 637 (CVD-Verfahren) beschrieben. Al₂O₃-Plättchen sind beispielsweise aus der EP 0 763 573 A1 bekannt. Plättchenförmige Substrate, die mit ein oder mehreren seltenen Erdmetallsulfiden belegt sind, werden z. B. in der DE OS 198 10 317 offenbart.

Weiterhin geeignet sind Metalleffektpigmente, insbesondere für wässrige oder lösemittelhaltige Systeme modifizierte Aluminiumplätttchen, wie sie von der Fa. Eckart unter der Marke Rotovario Aqua® oder Stapa Hydroxal® für wässrige Anwendungen vertrieben werden, sowie Variocrom®- und Paliocrom®-Pigmente der Fa. BASF, insbesondere auch solche aus den Offenlegungsschriften EP 0 681 009 A1, EP 0 632 110 A1, EP 0 634 458 A1, sowie LCP-Pigmente (Liquid Crystal Polymers). Weiterhin geeignet sind ebenfalls alle dem Fachmann bekannten holographischen Pigmente sowie plättchenförmigen Pigmente, die Metallschichten aufweisen. Derartige Pigmente werden von der Fa. Flex, Fa. BASF, der Fa. Eckart und der Fa. Schlenk vertrieben.

Die erfindungsgemäßen Pigmentpräparationen können ein oder mehrere Glanzpigmente enthalten, vielfach können durch die Verwendung von mindestens zwei verschiedenen Glanzpigmenten besondere Farb- und Glanzeffekte erzielt werden.

Zusätzlich zu den Perlglanzpigmenten und BiOCI-Pigmenten kann die erfindungsgemäße Pigmentzubereitung auch noch Rußteilchen, Fluoreszenz- und/oder organische Buntpigmente enthalten. Vorzugsweise besteht die Zubereitung dann aus 50-100 Gew.% an BiOCI- und Glanzpigmenten und 0-50 Gew.% an Rußteilchen, Fluoreszenz- und/oder Buntpigmenten. Der Gehalt aller Pigmente in der Pigmentpräparation sollte dabei aber 99,4 Gew.% nicht überschreiten. Weiterhin können solche Substanzen und Partikel (Racer) zugesetzt werden, die eine Produktidentifizierung ermöglichen.

Die Verwendung von sphärischen Teilchen in Offset-Druckfarben ist bereits aus der JP 62-143 984 bekannt. Die dort beschriebenen sphärischen Partikel aus Harz oder Wachs mit einem Gehalt von 0,1-20 Gew.% in die Druckfarbe, die weder Glanz- noch BiOCI-Pigmente enthält, eingerührt, um die Aufbaueigenschaften zu verbessern und das Rupfen des Papiers vom Gummituch zu verhindern.

Geeignete sphärische Materialien sind insbesondere Glas-, Wachs- oder Polymerhohlkugeln aus Vinylharzen, Nylon, Silicon, Epoxydharzen, Olefinharzen, Polystyrolen sowie anorganische Materialien, wie z. B. TiO₂, SiO₂ oder ZrO₂. Vorzugsweise werden Hohlkugeln, ferner auch Vollkugeln, mit einer Teilchengröße von 0,05 bis 150 µm verwendet. Insbesondere bevorzugt werden in der erfindungsgemäßen Pigmentpräparation Glas-, Wachs- oder Polymerhohlkugeln eingesetzt.

Die sphärischen Teilchen besitzen eine Teilchengröße von 0,05-150 µm, vorzugsweise 0,05-50 µm, und insbesondere von 1-30 µm. Sphärische Teilchen auf Basis von SiO₂ in einem Teilchenbereich von 3-10 µm sind z. B. als Materialien für die Hochdruckflüssigkeitschromatographie bekannt und werden z. B. als LiChrospher® von der Fa. Merck KGaA, Darmstadt vertrieben. Vorzugsweise werden solche Materialien in monodisperser Form, d. h. mit einer möglichst einheitlichen Teilchengröße, eingesetzt. Bekannt sind solche monodispersen sphärischen Teilchen auf Basis von SiO₂, TiO₂ und ZrO₂. Monodisperses SiO₂ kann beispielsweise nach der DE 36 16 133 hergestellt werden. Glashohlkugeln werden beispielsweise unter dem Handelsnamen Q-CEL von der Fa. PQ Corporation, USA oder Scotchlite von der Fa. 3M, Frankfurt, Deutschland vertrieben.

Die verbesserte Deagglomeration der BiOCI- und Glanzpigmente in einer Druckfarbe zeigt sich bereits mit geringen Mengen an sphärischen Teilchen in der Pigmentzubereitung. So werden bereits bei einem Gehalt von 0,5 Gew.% an sphärischen Teilchen bezogen auf die Pigmentpräparation deutlich verbesserte Fortdruckeigenschaften bei den Druckfarben, die die erfindungsgemäße Pigmentpräparation enthalten, festgestellt. In der Regel werden Pigmentpräparationen mit einem Gehalt von 1-10 Gew.%, insbesondere 1-5 Gew.% an sphärischen Teilchen verwendet.

Bei der erfindungsgemäßen Pigmentzubereitung wird durch die sphärischen Teilchen verhindert, dass die plättchenförmigen BiOCI- und Glanzpigmente sich in nennenswertem Umfang aufeinanderlegen und damit eine starke Adhäsion ausüben können. Wie elektronenmikroskopische Aufnahmen zeigen, lagern sich die sphärischen Teilchen an der Oberfläche des plättchenförmigen Glanzpigmentes an und ermöglichen daher wie eine Art Kugellager die leichte Verschiebbarkeit der Pigmentplättchen in der Druckfarbe gegeneinander, wodurch ein Aufbauen und Pelzen der Pigmente während des Druckvorganges weitgehend unterbunden wird.

Bei der Verwendung der erfindungsgemäßen Pigmentpräparation in Druckfarben können die sphärischen Teilchen schon bestehende Pigmentagglomerate "zerschlagen", indem die sphärischen Teilchen beim Druckvorgang in die Pigmenthäufungen gepresst werden und diese Verbunde "sprengen". Die größeren sphärischen Teilchen können zum Teil dabei zerstört werden. Im Druckerzeugnis findet man vorzugsweise nur noch Teilchen mit einem kleineren Teilchendurchmesser, während die Bruchstücke lockernd im Pigmentverbund wirken.

Als weiteren Bestandteil kann die erfindungsgemäße Pigmentzubereitung ein Phosphatderivat in Mengen von 0,1-5 Gew.%, vorzugsweise 1-3 Gew.%, bezogen auf die Pigmentpräparation enthalten. Geeignete Phosphatderivate sind z. B. die höheren und niedrigeren Metapolyphosphate sowie Pyrophosphate. Besonders bevorzugt sind die Alkalimetallpolyphosphate, insbesondere das Natriumpolyphosphat.

Häufig empfiehlt es sich in die Pigmentzubereitung noch ein Dispergiermittel einzurühren. Alle dem Fachmann bekannten Dispergiermittel können verwendet werden, z. B. wie sie in Karsten, Lackrohstofftabellen, 9. Auflage 1992, beschrieben werden. Insbesondere geeignet sind Dispergiermittel auf Basis von Polyacrylaten oder Polymethacrylaten. Die eingesetzte Menge an Dispergiermittel sollte nicht mehr als 10 Gew.% betragen, vorzugsweise 0,05-5 Gew.%, bezogen auf die fertige Druckfarbe.

Die Herstellung der Pigmentzubereitung ist einfach und leicht zu handhaben. Zunächst wird das Glanzpigment mit den sphärischen Teilchen durch Schütteln oder in einem Trockenmischer intensiv gemischt. Anschließend erfolgt die Zugabe des Phosphatderivates und der BiOCI-Pigmente, die entweder als Pulver eingerührt oder in Form einer Dispersion bestehend aus Salz und einer flüssigen Komponente zugegeben werden. Als flüssige Komponente sind insbesondere Mineralöle oder andere nicht trocknende Öle, geeignet, ferner trocknende Öle, wie z. B. Leinöl, Sojaöl, sowie Wasser oder organische Lösemittel. Die flüssige Komponente sollte dann maximal 10 Gew.% bezogen auf die Pigmentpräparation betragen. Die Pigmentpräparation kann auch hergestellt werden, indem man alle Komponenten gleichzeitig vorlegt und dann intensiv miteinander mischt. Die fertige Pigmentzubereitung kann dann Formulierungen, wie z. B. Druckfarben, Lacken, Farben, Kunststoffen und kosmetischen Zubereitungen, zugemischt werden.

Die erfindungsgemäße Pigmentzubereitung eignet sich insbesondere zur Pigmentierung von Druckfarben. Derartige pigmentierte Druckfarben können für alle bekannten Drucktechniken, insbesondere für Offsetdruck, ferner Hochdruck, Letterset, Tiefdruck, Flexodruck, Siebdruck, weiterhin für die Überdrucklackierung als auch für Beschichtungen eingesetzt werden. Bevorzugt werden sie für den Offsetdruck verwendet. Dabei kann sie sowohl für den Rollenoffsetdruck als auch für den Bogenoffsetdruck im Trocken- und Nassverfahren verwendet werden; insbesondere geeignet ist sie jedoch für den Bogenoffsetdruck im Nassverfahren.

Die Dispergierung der erfindungsgemäßen Pigmentpräparation in die Druckfarbe bzw. in das Bindemittel erfolgt dann mit Hilfe eines Rührwerks mit Propeller- oder Flügelrührer, gegebenenfalls bei unterschiedlichen Dispergiertemperaturen. Dabei werden die Pigmentteilchen vom Bindemittel umhüllt.

Für die Herstellung einer pigmentierten Druckfarbe, insbesondere einer Offset-Druckfarbe, können alle handelsüblichen Bindemittel verwendet werden. Derartige Bindemittel bestehen aus bekannten synthetischen oder natürlichen Harzen, gegebenenfalls trocknenden Ölen, Mineralölen und Additiven, wie sie beispielsweise in Karsten, Lackrohstofftabellen, 9. Auflage 1992, beschrieben werden. Vorzugsweise besitzen die verwendeten Harze eine relativ niedrige Schmelz- bzw. Lösemittelviskosität. Aber auch hochpolymerisierte und hochviskose Anteile können enthalten sein. Als besonders geeignet haben sich Kombinationen aus Hartharzen und Alkydharzen erwiesen, da diese die Pigmente besser benetzen und glänzendere sowie reibechtere Drucke ergeben. Insbesondere werden Bindemittel verwendet, die aus 50-90 Gew.% Hartharz und 5-50 Gew.% Alkydharz zusammengesetzt sind. Bei den verwendeten Hartharzen handelt es sich vorzugsweise um Kohlenwasserstoffharze. Die verwendeten Kohlenwasserstoffharze können eine Säurezahl nahe 0 mg KOH/g Substanz haben, sie können aber auch modifiziert sein und Säurezahlen bis 30 mg KOH/g Substanz aufweisen. Weiterhin kann das Bindemittel 1-50 Gew.% eines Mineralöls enthalten. Die Farbanteile sind in einer Weise aufeinander abgestimmt, dass eine für die niedrigere Farbviskosität geeignete stabile Farb/Wasser-Balance erreicht wird.

Die Trocknung der Druckfarbe erfolgt durch oxidative Polymerisation der Harze und durch Öle, wie z. B. Lein-, Holz- oder Rizinusöl, die beim Drucken in das Papier wegschlagen. Der Trocknungsvorgang kann durch Zusätze von Trocknungskatalysatoren (Sikkative), meist fettsaure Salze von Kobalt, Blei, Mangan, usw., weiter beschleunigt werden. Der Anteil an trocknenden Ölen in den erfindungsgemäßen Offset-Druckfarben liegt im Bereich von 0-50 Gew.%, vorzugsweise bei 0-30 Gew.%. Weitere Additive können in die Druckfarbe eingebracht werden, um die Farbeigenschaften für spezielle Anwendungen zu verändern. Diese Zusätze können Wachsverbindungen, Trocknungsmittel, Dispersionsmittel, Lösemittel, Verdicker, Gleitmittel, Pigmentfüllstoffe und/oder Antioxidantien sein. Weitere Details zu den grundsätzlichen Eigenschaften von Offset-Druckfarben finden sich in A. Rosenberg, Der Polygraph (11), 1153 (1987) bzw. B. Grande, Coating (4), 138 (1987).

Häufig ist bei den sehr niedrigviskosen pigmenthaltigen Druckfarben keine ausreichende Punktschärfe gegeben. Diese ist jedoch gerade in feinen Rastern erforderlich um Zusetzen des Drucks zu vermeiden. Es empfiehlt sich daher die Druckfarbe mit Struktur zu versehen. So kann die Zugabe eines Strukturbildners die Punktschärfe ausreichend verbessern. Vorzugsweise enthält die erfindungsgemäße Pigmentpräparation dann 0,1-3 Gew.% eines Strukturbildners. Neben der verbesserten Punktschärfe zeigt eine derartig modifizierte Pigmentpräparation in einer Druckfarbe eine deutlich bessere Pigmentübertragung, bessere Fortdruckeigenschaften und eine Verbesserung des Absetzverhaltens.

Durch die Wahl der Harze in den Bindemitteln und den Anteil an BiOCIund Glanzpigmenten in der Druckfarbe können die für den Druckprozess ausschlaggebenden Parameter wie Dispergierbarkeit, Zügigkeit und Viskosität individuell eingestellt werden. Die Viskosität und die Zügigkeit sind bei gleichem Farbaufbau voneinander abhängig, können aber durch speziellen Farbaufbau auch gezielt einzeln verändert werden. Hierbei ist zu beachten, dass Druckfarben mit einem zu hohen Grad an Zügigkeit verursachen können, dass Teile des Papiers reißen (Rupfen). Farben mit ungenügender Zügigkeit werden nicht in geeigneter Weise bei dem Druckvorgang übertragen. Wenn das Eindringen der Farbe zu groß ist, wird die Farbe auf der entgegengesetzten Seite des Papiers sichtbar oder verursacht eine Fleckigkeit oder Undeutlichkeit der Abbildung. Eine schlecht kontrollierte Eindringung kann Verschmieren und Ablegen verursachen. Die Druckfarbe zeigt dabei keinerlei Probleme mit dem Abspritzen von den Farbwalzwerken, auch nicht bei Druckgeschwindigkeiten von 10000 Bögen pro Stunde.

Druckfarben enthaltend die erfindungsgemäße Pigmentzubereitung sind insbesondere in Hinblick auf graphische Erzeugnisse der Werbebranche und bei hochwertigen Druckerzeugnissen von besonderer Bedeutung, da die fertigen Drucke aufgrund ihres Glanzes ästhetisch höchste Ansprüche erfüllen.

Zur Verbesserung der Druckgeschwindigkeiten und Fortdruckeigenschaften empfiehlt es sich, insbesondere beim Offsetdruck, Drucktücher mit einer glatten Oberfläche zu verwenden, wobei die modifizierte Oberfläche vorzugsweise aus Polyvinylchlorid, Polyurethan, Polyester oder Teflon besteht und eine Oberflächenhärte von ca. 88-95° Shore aufweisen sollte. Die Verwendung eines solchen Drucktuchs in Kombination mit der erfindungsgemäßen Pigmentpräparation in der Druckfarbe gewährleistet einen einwandfreien Farbübertrag.

Gegenstand der Erfindung sind somit auch Druckfarben, die bis zu 80 Gew.%, vorzugsweise 5 bis 70 Gew.%, insbesondere 10 bis 55 Gew.%, der erfindungsgemäßen Pigmentpräparation enthalten.

Die erfindungsgemäße Pigmentpräparation zeichnet sich durch ihren hohen Glanz aus und kann daher für die verschiedensten Zwecke eingesetzt werden. Neben der Verwendung in Druckfarben, einschließlich Sicherheits-Druckfarben, kann sie weiterhin in Kunststoffen, Farben, Lacken und aufgrund des guten Skin-Feelings auch in kosmetischen Zubereitungen Anwendung finden.

Gegenstand der Erfindung sind somit auch Formulierungen, die die erfindungsgemäße Pigmentpräparation enthalten.

Die folgenden Beispiele sollen die Erfindung näher erläutern, ohne sie jedoch zu begrenzen.

### Beispiele

### I. Pigmentpräparation

### Beispiel 1

600,0 g Iriodin® 123 (Perlglanzpigment: TiO₂ auf Glimmer der Teilchengröße 5-20 µm der Fa. Merck KGaA) werden mit 27 g Scotchlite S22 Fa. 3M versetzt und bei 300 Upm homogen eingerührt. Als Rührermotor dient ein Dispermat AE 6C. Anschließend wird eine Suspension aus 28,4 g Natriumpolyphosphat fein dispergiert in 28,4 g Mineralöl PKWF 28/31 hergestellt und zusammen mit 428,6 g Bi-Flair® 66a (BiOCI-Pigment der Teilchengröße 1 - 20 µm der Fa. Merck KGaA) gemischt. Diese wird zu dem Iriodin® 123 / Scotchlite-Gemisch zugegeben. Das Gemenge wird 16 min bei 650 Upm homogen gemischt.

### Beispiel 2

600,0 g Iriodin® 326 (Perlglanzpigment: TiO₂/Fe₂O₃ auf Glimmer der Teilchengröße 5-25 µm der Fa. Merck KGaA) werden mit 22,5 g Scotchlite S 22 versetzt und bei 300 Upm homogen eingerührt. Als Rührermotor dient ein Dispermat AE 6C. Als Rührer wird ein Propellerrührer (Durchmesser 16 cm) verwendet. Anschließend werden zwei Suspensionen hergestellt.
1. 22,5 g Natriumpolyphosphat werden in 23,7 g Mineralöl mit einer Perlmühle dispergiert.
2. 15,0 g PV Echt Gelb (Fa. Hoechst) werden in 85 g Mineralöl PKWF 28/31 ebenfalls mit einer Perlmühle dispergiert.

Die beiden Suspensionen werden mit 214 g Bi-Flair® 66a (Anteigungen von BiOCI-Pigmenten in Mineralöl) homogen vermischt und zu dem Iriodin® 326 / Scotchlite-Gemisch zugegeben. Das Gemisch wird 16 min bei 650 Upm homogen gemischt.

### II. Herstellung einer Offsetdruckfarbe

2.1 489 g der Pigmentpräparation aus Beispiel 1 werden in 611 g Gebr. Schmidt Bronzefirnis 11A1034-1 (Fa. Gebr. Schmidt) mit einem Rührer (Durchmesser 10 cm) bei 450 Upm 20 min eingerührt. Die Farbe kann dann direkt an der Druckmaschine verdruckt werden.
2.2 655 g der Pigmentpräparation von Beispiel 2 werden in 845 g Gebr. Schmidt Bronzefirnis 11A1034-1 mit einem Rührer (Durchmesser 10 m) bei 45 Upm 20 min eingerührt. Die Farbe kann dann direkt an der Druckmaschine verdruckt werden.

## Patentansprüche

1. Pigmentpräparation enthaltend BiOCI-Pigmente, ein oder mehrere Glanzpigmente, sphärische Teilchen und gegebenenfalls Additive aus dem Druckbereich.

2. Pigmentpräparation nach Anspruch 1, **dadurch gekennzeichnet, dass** das Verhältnis BiOCI zu Glanzpigment 99 : 1 bis 1 : 99 beträgt.

3. Pigmentpräparation nach Anspruch 1 oder 2, **dadurch gekennzeichnet, dass** das Glanzpigment ein Perlglanzpigment ist.

4. Pigmentpräparation nach einer der Ansprüche 1 bis 3, **dadurch gekennzeichnet, dass** das Perlglanzpigment ein mit ein oder mehreren Metalloxidschichten belegtes Glimmer-, SiO₂, Glas- oder Al₂O₃-Plättchen ist.

5. Pigmentpräparation nach einem der Ansprüche 1 bis 4, **dadurch gekennzeichnet, dass** die sphärischen Teilchen Glas-, Wachs-, SiO₂-Kugeln oder Polymerhohlkugeln sind.

6. Pigmentpräparation nach einem der Ansprüche 1 bis 5, **dadurch gekennzeichnet, dass** sie zusätzlich bis zu 30 Gew.% einer flüssigen Komponente enthält.

7. Pigmentpräparation nach einem der Ansprüche 1 bis 6, **dadurch gekennzeichnet, dass** sie zusätzlich ein Phosphatderivat enthält.

8. Pigmentpräparation nach Anspruch 7, **dadurch gekennzeichnet, dass** das Phosphatderivat in Mengen von 0,1-5 Gew.% enthalten ist.

9. Verwendung der Pigmentpräparation nach Anspruch 1 in Druckfarben, Sicherheitsdruckfarben, Lacken, Farben, Kunststoffen und in kosmetischen Formulierungen.

10. Druckfarben enthaltend bis zu 80 Gew.% der Pigmentpräparation nach Anspruch 1.

11. Formulierungen enthaltend die Pigmentpräparation nach Anspruch 1.
